# EUROPÄISCHE PATENTSCHRIFT

(11) **EP 4 077 253 B1**
(45) Veröffentlichungstag und Bekanntmachung des Hinweises auf die Patenterteilung: **12.06.2024**
(21) Anmeldenummer: 20829876.0
(22) Anmeldetag: 16.12.2020
(51) Int. Cl.: C07C 29/70, C07C 31/10, C07C 31/12, C07C 31/125

(54) **VERFAHREN ZUR HERSTELLUNG VON METALLALKOHOLATEN**
METHOD FOR PRODUCING METAL ALCOHOLATES
PROCÉDÉ DE PRODUCTION D'ALCOOLATES MÉTALLIQUES

(30) Priorität: 18.12.2019 EP 19217475
(43) Veröffentlichungstag der Anmeldung: 26.10.2022
(73) Patentinhaber: BASF SE, 67056 Ludwigshafen am Rhein (DE)
(72) Erfinder: WEISSKER, Wolf-Steffen, 67056 Ludwigshafen (DE); HUGUET SUBIELA, Nuria, 67056 Ludwigshafen (DE)
(74) Vertreter: BASF IP Association
(86) Internationale Anmeldenummer: PCT/EP2020/086362
(87) Internationale Veröffentlichungsnummer: WO 2021/122702

(56) Entgegenhaltungen:
- EP-A1- 0 776 995
- DE-A1- 2 726 491

## Beschreibung

Metallalkoholate werden als starke Basen in der Synthese zahlreicher Chemikalien eingesetzt, beispielsweise bei der Herstellung von Wirkstoffen für die Pharma- bzw. Agrarindustrie. Metallalkoholate finden außerdem Anwendung als Katalysatoren in Umesterungs- und Amidierungsreaktionen.

Metallalkoholate (MOR) können gemäß folgender Reaktionsgleichung aus Metallhydroxiden (MOH) und Alkoholen (ROH), die in einer Reaktivdestillationskolonne im Gegenstrom geführt werden, hergestellt werden, wobei bei der Reaktion anfallendes Wasser mit dem Destillat entfernt wird:

MOH + ROH MOR + H₂O

Die EP 0 091 425 A1 beschreibt ein solches Verfahren, wobei ein wässriges Alkalimetallhydroxid in den Verstärkungsteil und ein gasförmiger Alkohol in den Sumpf der Kolonne eingeführt wird. Wasser wird über den Kopf der Kolonne als Azeotrop ausgeführt und einer Auftrennung unterzogen. Ähnliche Verfahren sind in der EP 1 997 794 A1 und der DD 246 988 A1 beschrieben.

Metallalkoholate können auch mittels Umalkoholisieren gemäß folgender Formel hergestellt werden, wobei Alkoholate niedrigsiedender Alkohole (MOR¹) mit einem höhersiedenden Alkohol (R²OH) mittels Reaktivdestillation zu Alkoholaten höhersiedender Alkohole und niederen Alkoholen umgesetzt werden:

MOR¹ + R²OH MOR² + R¹OH

Ein solches Verfahren ist beispielsweise in der DE 27 26 491 sowie der DE 1 254 612 beschrieben. Dabei wird ein Gemisch des höhersiedenden Alkohols und des Alkoholats des niedrigsiedenden Alkohols in die Kolonne eingespeist.

Die bekannten Verfahren zur Herstellung von Metallalkoholaten höhersiedender Alkohole, wie Isopropanol oder tert-Butanol, weisen einen hohen Energieaufwand auf. Die Aufgabe der vorliegenden Erfindung liegt in der Bereitstellung eines Verfahrens zur Herstellung von Metallalkoholaten mit vermindertem Energiebedarf.

Die Aufgabe wird gelöst durch ein Verfahren zur Herstellung von Metallalkoholaten mittels Umalkoholisieren, wobei man
- ein niederes Metallalkoholat über einen seitlichen Zulauf in eine Reaktivdestillationskolonne mit oberhalb des Zulaufs gelegenem Verstärkungsteil und unterhalb des Zulaufs gelegenem Abtriebsteil einspeist;
- einen höheren Alkohol in den Abtriebsteil, den Sumpf und/oder einen Sumpfumlauf der Kolonne einspeist;
- am Sumpf der Kolonne und/oder aus dem Sumpfumlauf, vorzugsweise nach dem Verdampfer, eine Lösung eines höheren Metallalkoholats im höheren Alkohol abzieht; und
- am Kopf der Kolonne einen niederen Alkohol umfassenden Brüden abzieht, den Brüden zumindest teilweise kondensiert und einen Teilstrom des Kondensats als Rücklauf auf den Kopf der Kolonne zurückführt;
wobei der höhere Alkohol einen höheren Siedepunkt aufweist als der niedere Alkohol.

Im erfindungsgemäßen Verfahren werden das niedere Metallalkoholat und der höhere Alkohol umalkoholisiert, um das höhere Metallalkoholat und den niederen Alkohol zu erhalten. Am Kopf der Reaktivdestillationskolonne wird ein niederen Alkohol umfassender Brüden und am Sumpf der Kolonne und/oder aus dem Sumpfauslauf eine Lösung des höheren Metallalkoholats im höheren Alkohol abgezogen. Es werden also eine gasförmige und eine flüssige Fraktion abgezogen. Der Reinheitsgrad der erhaltenen Fraktionen ist entscheidend für die Effizienz des Verfahrens und die Verwertbarkeit der Fraktionen.

Der am Kopf der Kolonne abgezogene Brüden wird zumindest teilweise kondensiert und ein Teilstrom des erhaltenen Kondensats wird als Rücklauf auf den Kopf der Kolonne zurückgeführt. In der Kolonne stehen eine Gasphase und eine flüssige Phase miteinander in Kontakt, die im Gegenstrom zueinander geführt werden. Es findet ein Stoffaustausch zwischen der Gasphase und der flüssigen Phase statt. Dabei reichern sich die leichter flüchtigen Anteile in Richtung des Kopfs der Kolonne in der Gasphase und die schwerer flüchtigen Anteile in Richtung des Sumpfs der Kolonne in der flüssigen Phase an.

Der Reinheitsgrad insbesondere der am Sumpf und/oder aus dem Sumpfumlauf abgezogenen Lösung des höheren Metallalkoholats kann unter anderem über das Rücklaufverhältnis am Kopf der Kolonne eingestellt werden. In der Regel bedingt ein größeres Rücklaufverhältnis einen höheren Reinheitsgrad der Lösung des höheren Metallalkoholats.

Es wurde gefunden, dass das Einspeisen des höheren Alkohols unterhalb des Zulaufs des niederen Metallalkoholats eine signifikant niedrigere Kontamination der Lösung des höheren Metallalkoholats mit niederem Alkohol bedingt. Um die gewünschten Spezifikationen zu erreichen, wird im erfindungsgemäßen Verfahren also ein niedrigeres Rücklaufverhältnis und ein verringerter Sumpfumlauf benötigt. Vorteilhafterweise weist das erfindungsgemäße Verfahren bei gleicher Trennaufgabe somit einen signifikant niedrigeren Energiebedarf auf.

Die Bezeichnung "niederer" und "höherer" Alkohol bedeutet niedriger siedender Alkohol und höher siedender Alkohol. Es versteht sich, dass im erfindungsgemäßen Verfahren der höhere Alkohol ("Umalkoholisierungsalkohol") einen höheren Siedepunkt aufweist als der Alkohol des niederen Metallalkoholats. Das erfindungsgemäße Verfahren ist allgemein anwendbar auf die Umsetzung von Metallalkoholaten niederer Alkohole mit höheren Alkoholen, soweit eine Destillierbarkeit der Alkohole gegeben ist und das Löslichkeitsverhalten der niederen und höheren Metallalkoholate die Umsetzung in flüssiger Phase zulassen.

Das niedere Metallalkoholat wird über einen seitlichen Zulauf in die Reaktivdestillationskolonne eingespeist. Unter der Bezeichnung "seitlich" wird verstanden, dass die Einspeisung unterhalb des Kolonnenkopfes und oberhalb des Kolonnensumpfes erfolgt. Der Ort des Zulaufs des niederen Metallalkoholats trennt die Kolonne in einen Verstärkungsteil (oberhalb des Zulaufs) und einen Abtriebsteil (unterhalb des Orts des Zulaufs). Die Umalkoholisierung findet im Abtriebsteil der Kolonne statt.

Üblicherweise speist man das niedere Metallalkoholat als Lösung in dem niederen Alkohol ein, wobei die Lösung 20 bis 40 Gew.-% des niederen Metallalkoholats umfasst, bevorzugt 25 bis 35 Gew.-% des niederen Metallalkoholats, wie 28 bis 32 Gew.-% des niederen Metallalkoholats, bezogen auf das Gesamtgewicht der Lösung des niederen Metallalkoholats.

Das niedere Metallalkoholat ist üblicherweise ein Metallalkoholat eines einwertigen C₁-C₄-Alkohols, also Methanolat, Ethanolat, Propanolat, Butanolat und deren Konstitutionsisomeren, einschließlich cyclischer Alkoholate. Besonders bevorzugt ist das niedere Metallalkoholat Metallmethanolat oder Metallethanolat, insbesondere Metallmethanolat.

Das niedere Metallalkoholat ist üblicherweise ein Alkalimetallalkoholat, bevorzugt ein Natriumalkoholat oder ein Kaliumalkoholat.

Besonders bevorzugt ist das niedere Metallalkoholat Natriummethanolat oder Kaliummethanolat, insbesondere Natriummethanolat.

Die Temperatur der Lösung des niederen Metallalkoholats am seitlichen Zulauf wird geeigneter Weise so gewählt, dass das niedere Metallalkoholat stets in Lösung bleibt. Die Temperatur der Lösung des niederen Metallalkoholats beträgt vorzugsweise mindestens 6 °C, insbesondere mindestens 10 °C und besonders bevorzugt mindestens 20 °C, beispielsweise 25 °C oder 30 °C. Die Lösung des niederen Metallalkoholats kann bis zur Siedetemperatur vorgeheizt werden, beispielsweise in einem Wärmetauscher, in dem gleichzeitig die am Sumpf der Kolonne abgezogene Lösung des höheren Metallalkoholats abgekühlt wird. Dies ist energetisch besonders vorteilhaft.

In den Abtriebsteil, den Sumpf und/oder einen Sumpfumlauf der Reaktivdestillationskolonne wird ein höherer Alkohol eingespeist, vorzugsweise in den Sumpf und/oder den Sumpfumlauf. Der höhere Alkohol kann in flüssiger Form oder gasförmig in den Abtriebsteil, den Sumpf oder Sumpfumlauf der Kolonne eingespeist werden. Vorzugsweise wird der höhere Alkohol in flüssiger Form in den Abtriebsteil, den Sumpf und/oder Sumpfumlauf der Kolonne eingespeist. Besonders bevorzugt wird der höhere Alkohol in flüssiger Form in den Sumpf und/oder Sumpfumlauf, insbesondere den Sumpfumlauf der Kolonne eingespeist.

Die Reaktivdestillationskolonne weist üblicherweise einen Verdampfer auf, vorzugsweise einen Umlaufverdampfer. Im Verdampfer wird ein Teilstrom der am Sumpf der Kolonne abgezogenen Lösung des höhersiedenden Metallalkoholats erhitzt und zumindest teilweise gasförmig zurück in den Sumpf der Kolonne geführt. Alternativ oder zusätzlich wird der Sumpf direkt beheizt. Ein Teil des höheren Alkohols liegt in der Reaktivdestillationskolonne gasförmig vor und steigt Richtung Kopf der Kolonne auf.

Der höhere Alkohol ist üblicherweise ausgewählt unter einwertigen C₂-C₁₀-Alkoholen, also Ethanol, Propanol, Butanol, Pentanol, Hexanol, Heptanol, Octanol, Nonanol, Decanol und deren Konstitutionsisomeren, einschließlich cyclischer Alkohole. Grundsätzlich können auch mehrwertige C₂-C₁₀-Alkohole wie Diole, beispielsweise Ethylenglycol, Diethylenglycol, Propandiol oder Butandiol, verwendet werden. Besonders bevorzugt ist der höhere Alkohol ausgewählt unter Isopropanol, sec-Butanol, 2-Methyl-2-butanol, tert-Butanol, 2-Methyl-2-pentanol, 3-Methyl-3-pentanol, 3-Ethyl-3-pentanol, 2-Methyl-2-hexanol und 3-Methyl-3-hexanol. Ganz besonders bevorzugt sind Isopropanol, 2-Methyl-2-butanol, 3-Methyl-3-pentanol und 3-Ethyl-3-pentanol, insbesondere Isopropanol und 2-Methyl-2-butanol.

Am Sumpf der Kolonne und/oder aus dem Sumpfumlauf wird eine Lösung des höheren Metallalkoholats im höheren Alkohol abgezogen. Die abgezogene Lösung des höheren Metallalkoholats weist vorteilhafterweise nur geringe Mengen an niederem Alkohol auf, was ein effizientes Verfahren erlaubt. Bevorzugt umfasst die Lösung des höheren Metallalkoholats höchstens 1,0 Gew.-% des niederen Alkohols, besonders bevorzugt höchstens 0,5 Gew.-% des niederen Alkohols und ganz besonders bevorzugt höchstens 0,3 Gew.-% des niederen Alkohols, beispielsweise 0,01 bis 0,20 Gew.-% des niederen Alkohols oder 0,01 bis 0,10 Gew.-% des niederen Alkohols, bezogen auf das Gesamtgewicht abgezogenen Lösung des höheren Metallalkoholats. Die Methanol-Konzentration in der Lösung des höheren Metallalkoholats kann beispielsweise mittels Dampfraumanalyse oder mittels Gaschromatographie erfolgen.

Die Lösung des höheren Metallalkoholats umfasst üblicherweise 3 bis 60 Gew.-% des höheren Metallalkoholats, bevorzugt 5 bis 55 Gew.-% und besonders bevorzugt 7 bis 50 Gew.-% des höheren Metallalkoholats, beispielsweise 7 bis 30 Gew.-%, 7 bis 25 Gew.-%, 7 bis 20 Gew.-% oder 7 bis 15 Gew.-% des höheren Metallalkoholats, bezogen auf das Gesamtgewicht der abgezogenen Lösung des höheren Metallalkoholats. Die Konzentration des höheren Metallalkoholats in der Lösung des höheren Metallalkoholats kann beispielsweise mittels Titration bestimmt werden.

Am Kopf der Kolonne wird ein niederen Alkohol umfassender Brüden abgezogen. Der abgezogene Brüden besteht im Wesentlichen aus niederem Alkohol und weist vorteilhafterweise nur geringe Mengen an höherem Alkohol auf, was ein effizientes Verfahren erlaubt. Der Brüden wird zumindest teilweise kondensiert ("Kopfkondensat"), wobei das Kopfkondensat bevorzugt höchstens 1,0 Gew.-% des höheren Alkohols, besonders bevorzugt höchstens 0,6 Gew.-% des höheren Alkohols und ganz besonders bevorzugt höchstens 0,5 Gew.-% des höheren Alkohols, umfasst, bezogen auf das Gesamtgewicht des Kopfkondensats. Die Konzentration des höheren Alkohols im Kopfkondensat kann beispielsweise mittels Gaschromatographie bestimmt werden.

Das niedere Metallalkoholat und der höhere Alkohol werden in einer Reaktivdestillationskolonne miteinander in Kontakt gebracht. Das Inkontaktbringen des niederen Metallalkoholats und des höheren Alkohols erfolgt dabei im Gegenstrom. Der höhere Alkohol liegt teilweise gasförmig vor und steigt in der Kolonne Richtung Kopf der Kolonne auf, während die Lösung des niederen Metallalkoholats Richtung des Sumpfs der Kolonne absinkt. Durch den Kontakt von Gasphase und flüssiger Phase werden der höhere Alkohol und das niedere Metallalkoholat umalkoholisiert, um das höhere Metallalkoholat und den niederen Alkohol zu erhalten.

Als Reaktivdestillationskolonne kann eine übliche Reaktivdestillationskolonne verwendet werden. Die Kolonne ist beispielweise ausgewählt unter Füllkörper-, Packungs- und Bodenkolonnen, besonders bevorzugt Boden- und Packungskolonnen.

In geeigneten Bodenkolonnen sind Sieb-, Glocken- oder Ventilböden eingebaut, über welche die flüssige Phase strömt. Die Reaktivdestillationskolonne des erfindungsgemäßen Verfahrens weist vorzugsweise Böden als Einbauten auf, beispielsweise ausgewählt unter Glockenböden, Ventilböden, Tunnelböden und Thormann^{®}-Böden.

Füllkörperkolonnen können mit unterschiedlichen Formkörpern gefüllt werden. Wärme- und Stoffaustausch werden durch die Vergrößerung der Oberfläche aufgrund der meist etwa 25 bis 80 mm großen Formkörper verbessert. Bekannte Beispiele sind der Raschig-Ring (ein Hohlzylinder), Pall-Ring, Hiflow-Ring, Intalox-Sattel und dergleichen. Die Füllkörper können geordnet, aber auch regellos (als Schüttung) in die Kolonne eingebracht werden. Als Materialien kommen Glas, Keramik, Metall und Kunststoffe in Frage.

Strukturierte Packungen sind eine Weiterentwicklung der geordneten Füllkörper. Sie weisen eine regelmäßig geformte Struktur auf. Dadurch ist es bei Packungen möglich, Druckverluste bei der Gasströmung zu reduzieren. Es gibt verschiedene Ausführungen von Packungen, z. B. Gewebe- oder Blechpackungen.

Vorzugsweise umfasst der Verstärkungsteil der Kolonne Packungen, während der Abtriebsteil der Kolonne Böden umfasst.

Als Kopf der Kolonne wird der von Einbauten freie Bereich oberhalb des obersten Bodens bzw. oberhalb der obersten Packungsschicht bezeichnet. Er wird in der Regel von einem gewölbten Boden (Haube, z. B. Klöpperboden oder Korbbogenboden) gebildet, welcher das Abschlusselement der Reaktivdestillationskolonne bildet.

Als Sumpf der Kolonne wird der von Einbauten freie Bereich unterhalb des untersten Bodens bzw. unterhalb der untersten Packungsschicht bezeichnet.

Die geeignete Anzahl der theoretischen Trennstufen im Verstärkungsteil hängt von der Differenz der Dampfdrücke des höheren und des niederen Alkohols ab, wobei bei einer geringeren Differenz eine höhere Anzahl theoretischer Trennstufen vorteilhaft ist. Die geeignete Anzahl der theoretischen Trennstufen im Abtriebsteil hängt von der Differenz der Dampfdrücke des höheren und des niederen Alkohols sowie der Gleichgewichtslage der Reaktion ab, wobei eine höhere Anzahl theoretischer Trennstufen vorteilhaft ist, je stärker das Gleichgewicht auf Seiten der Edukte liegt. Die geeignete Anzahl der theoretischen Trennstufen sowohl im Verstärkungs- als auch im Abtriebsteil hängt außerdem vom zu erreichenden Reinheitsgrad von Sumpf- und Kopfprodukt und der verwendeten Rücklaufmenge ab, wobei eine höhere Anzahl theoretischer Trennstufen zum Erreichen einer höheren Reinheit bei gegebener Rücklaufmenge benötigt wird.

Die Kolonne kann aus mehreren kaskadenartig hintereinander geschalteten Einzelgefäßen bzw. Einzelkolonnen bestehen. Die Einzelgefäße bzw. Einzelkolonnen sind vorzugsweise übereinander angeordnet und können gegebenenfalls seitlich gegeneinander versetzt sein. In einer weiteren Ausführungsform sind die Einzelgefäße bzw. Einzelkolonnen unter Verwendung geeigneter Pumpeinrichtungen nebeneinander angeordnet.

In beiden Fällen muss durch entsprechende Verbindungsleitungen und gegebenenfalls Transporteinrichtungen sichergestellt werden, dass Abtriebs- und Verstärkungsteil direkt miteinander gekoppelt sind und sich der Rückfluss des Verstärkungsteils in vorteilhafter Weise auf die im Abtriebsteil stattfindende Umalkoholisierung auswirken kann. Bevorzugt sind der Reaktions- und der Verstärkungsteil in einer einzigen Kolonne übereinander angeordnet.

Der am Kopf der Kolonne abgezogene, niederen Alkohol umfassende Brüden wird zumindest teilweise kondensiert, wobei ein Kopfkondensat erhalten wird. Die Kondensation erfolgt vorzugsweise in einem oder mehreren in Serie geschalteten Platten- oder Rohrbündelkondensatoren oder Luftkühlern. Vorzugsweise verwendet man Luftkühler oder Rohrbündelkondensatoren oder Kombinationen davon. Die Kühlung der Kondensatoren kann je nach Bauart beispielsweise mittels Luft, Kühlwasser oder Sole erfolgen.

Vorzugsweise wird der Brüden im Wesentlichen vollständig kondensiert, beispielsweise zu mehr als 98 Gew.-% oder mehr als 99 Gew.-%, bezogen auf die Gesamtmenge des Brüdens.

Ein Teilstrom des erhaltenen Kondensats wird als Rücklauf auf den Kopf der Kolonne zurückführt. Das Rücklaufverhältnis beeinflusst die Reinheit der abgezogenen gasförmigen Fraktion und insbesondere der abgezogenen flüssigen Fraktion. Unter dem Rücklaufverhältnis wird das Verhältnis des Teilstroms des Kondensats (kg/h), der in die Kolonne zurückgeführt wird (Rücklauf), und des Teilstroms des Kondensats (kg/h), der aus dem Verfahren ausgeführt wird, verstanden.

Die genannten Rücklaufverhältnisse sind auf die Verwendung einwertiger, aliphatischer oder cycloaliphatischer höherer Alkohole gerichtet. Bei anderen höheren Alkoholen, z. B. bei zwei- oder mehrwertigen Alkoholen oder bei andern als aliphatischen oder cycloaliphatischen Alkoholen, kann die Rücklaufmenge gegebenenfalls höher sein. Die optimale Rücklaufmenge wird zweckmäßig in Vorversuchen ermittelt.

Das optimale Rücklaufverhältnis für das erfindungsgemäße Verfahren wird in bekannter Weise so festgelegt, dass bei einem wirtschaftlichen Optimum hinsichtlich der aufzuwendenden Leistung zur Trennung des im System vorhandenen niederen und höheren Alkohols ein Optimum hinsichtlich der Reinheit des am Kopf abzuführenden niederen Alkohols bzw. der am Sumpf abzuziehenden Lösung des höheren Alkoholats im höheren Alkohol erreicht wird.

Der am Kopf abgezogene niedere Alkohol, also der ausgeführte Teilstrom des Kopfkondensats, ist in der Regel von hoher Reinheit und kann ohne weitere Destillation wiederverwendet werden. Es wird nur der überschüssige Teil des Kopfkondensats aus dem System ausgeführt, der zur Einstellung der Rückflussmenge nicht benötigt wird. Der übrige Teil des Kopfkondensats wird als Rücklauf auf den Kopf der Kolonne zurückführt.

Die Reaktivdestillationskolonne weist üblicherweise einen Sumpfumlauf auf. In einer Ausführungsform wird dabei ein Teilstrom der am Sumpf der Kolonne abgezogenen Lösung des höheren Metallalkoholats im höheren Alkohol in den Sumpf der Kolonne zurückgeführt, während ein weiterer Teilstrom der am Sumpf der Kolonne abgezogenen Lösung des höheren Metallalkoholats im höheren Alkohol aus dem Verfahren ausgeführt wird. In einer weiteren Ausführungsform wird ein Teilstrom aus dem Sumpfumlauf in den Sumpf der Kolonne zurückgeführt, während ein weiterer Teilstrom aus dem Sumpfumlauf, vorzugsweise nach dem Verdampfer, entnommen und aus dem Verfahren ausgeführt wird.

Die Reaktivdestillationskolonne weist einen Verdampfer auf, der im Kolonnensumpf integriert ist, oder vorzugsweise einen in einen Sumpfumlauf aufgenommenen Verdampfer. Ein Teilstrom der am Sumpf der Kolonne abgezogenen Lösung des höheren Metallalkoholats wird über einen Sumpfumlauf dem Verdampfer zugeführt und anschließend als erhitzter, ggf. zwei-phasiger Fluidstrom in die Kolonne zurückgeführt. Geeignete Verdampfer sind z. B. Aufkocher, Naturumlaufverdampfer, Zwangsumlaufverdampfer und Zwangsumlaufentspannungsverdampfer.

Bei Zwangsumlaufverdampfern wird eine Pumpe verwendet, um die zu verdampfende Flüssigkeit durch den Erhitzer zu führen. Das erhaltene Dampf/Flüssigkeitsgemisch wird dann in die Kolonne zurückgeführt.

Bei Zwangsumlaufentspannungsverdampfern wird ebenfalls eine Pumpe verwendet, um die zu verdampfende Flüssigkeit durch den Erhitzer zu führen. Es wird ein überhitzter, flüssiger Rückführstrom erhalten, der in den Sumpf der Kolonne entspannt wird. Der Druck auf die aus der Kolonne abgezogene Lösung des Metallalkoholats, die zur Kolonne zurückgeführt wird, wird durch Überhitzen erhöht. Der überhitzte Rückführstrom wird über ein Durchflussbegrenzungsmittel entspannt. Hierdurch erfolgt eine Überhitzung der Flüssigkeit über ihren Siedepunkt in Bezug auf den Druck im Inneren der Kolonne.

Beim Durchtritt der überhitzten Flüssigkeit durch das Durchflussbegrenzungsmittel und Wiedereintritt in die Kolonne erfolgt eine schlagartige Verdampfung der Flüssigkeit. Diese schlagartige Verdampfung verläuft unter erheblicher Volumenvergrößerung und führt zu einer Beschleunigung des in die Kolonne eintretenden Fluidstroms. Vorteilhafterweise ist das Durchflussbegrenzungsmittel unmittelbar vor dem Wiedereintritt der überhitzten Flüssigkeit in die Kolonne, oder sogar im Inneren dieser, angeordnet.

Bevorzugt wird als Durchflussbegrenzungsmittel eine Blende, ein Ventil, eine Drossel, eine Lochscheibe, eine Düse, eine Kapillare oder Kombinationen davon, insbesondere ein Ventil, verwendet. Beispielsweise kann ein Drehkegelventil verwendet werden. Besonders bevorzugt ist es, wenn die Öffnungscharakteristik des Durchflussbegrenzungsmittels verstellbar ist. Auf diese Weise kann der Druck im Verdampfer auch bei geänderten Strömungsgeschwindigkeiten, wie sie beispielsweise bei An- und Abfahrvorgängen auftreten können, immer über dem Siededruck der Flüssigkeit, bezogen auf den Druck im Inneren der Kolonne, gehalten werden.

Vorteilhaft ist es, dass durch Betrieb des Verdampfers als Zwangsumlauf bzw. als Zwangsumlaufentspannung eine gegenüber Betrieb mit Naturumlauf erhöhte Strömungsgeschwindigkeit der Flüssigkeit in der Erhitzungsvorrichtung, z. B. im Rohrbündel des Wärmetauschers, erzielt wird. Durch die erhöhte Strömungsgeschwindigkeit besteht ein verbesserter Wärmeübergang zwischen Wärmetauscher und erhitzter Flüssigkeit, der wiederum zur Vermeidung lokaler Überhitzungen beiträgt.

Die bei Zwangsumlauf- bzw. Zwangsumlaufentspannungsverdampfern zu verwendende Pumpe wird bevorzugt zwischen der Entnahmeleitung und dem Verdampfer angeordnet.

In einer bevorzugten Ausführungsform weist die Kolonne einen Zwangsumlaufverdampfer auf und man speist den höheren Alkohol in flüssiger Form in den Zulauf zum Zwangsumlaufverdampfer ein.

Alternativ oder zusätzlich zum Sumpfumlaufverdampfer kann der Sumpf direkt beheizt werden, beispielsweise mittels eines Aufkochers.

Die Sumpftemperatur der Reaktivdestillationskolonne bestimmt bei gegebenem Druck die Konzentration des höheren Metallalkoholats in der am Sumpf der Kolonne oder aus dem Sumpfumlauf abgezogenen Lösung. Die Temperatur und damit die Konzentration werden zweckmäßigerweise so gewählt, dass das höhere Metallalkoholat im Sumpf stets in Lösung bleibt. Die Sumpftemperatur wird beispielsweise über einen Verdampfer und/oder eine direkte Beheizung des Sumpfs eingestellt.

Das erfindungsgemäße Verfahren kann sowohl unter Normaldruck als auch unter erhöhtem oder vermindertem Druck durchgeführt werden. Es ist vorteilhaft, die Reaktivdestillationskolonne bei einem Druck von 0,2 bis 10 bar absolut zu betreiben. Bevorzugt wird die Reaktivdestillationskolonne bei Umgebungsdruck, beispielsweise 1 bar absolut, betrieben.

Die Gleichgewichtslage der Reaktion ist bei der Herstellung mancher Alkoholate temperaturabhängig. In diesen Fällen können hohe Temperaturen vorteilhafterweise einen höheren Umsatz bedingen. Es kann außerdem vorteilhaft sein, das erfindungsgemäße Verfahren unter erhöhtem Druck durchzuführen, wie beispielsweise mindestens 1,5 bar absolut, mindestens 2,5 bar absolut, oder mindestens 5,0 bar absolut.

Das erfindungsgemäße Verfahren kann sowohl kontinuierlich als auch batchweise durchgeführt werden. Bevorzugt wird das erfindungsgemäße Verfahren kontinuierlich durchgeführt.

Innerhalb der Reaktivdestillationskolonne wird durch den ständigen Stoffaustausch und die sich ändernden Konzentrationen innerhalb der Gasphase und der flüssigen Phase das Reaktionsgleichgewicht ständig neu eingestellt, was einen hohen Umsatz ermöglicht. Beim Anfahren der Kolonne kann zur Einstellung der Menge des Rückflusses des niederen Alkohols dieser dem Kolonnenkopf zugeführt werden, wobei der Abtriebsteil und der Sumpf mit dem höheren Alkohol gefüllt sind. Außerdem kann dem höheren Alkohol niederer Alkohol zugesetzt werden.

Nach Erreichen der Betriebstemperatur wird dann die Lösung des niederen Metallalkoholats zugeführt. Während der Umsetzung bildet sich laufend neuer niederer Alkohol.

In einer weiteren Ausführungsform wird die Reaktivdestillationskolonne vor dem Anfahren mit dem höheren Alkohol befüllt und dieser zunächst auch als Rückfluss eingesetzt. Nach Erreichen der Betriebstemperatur wird dann die Lösung des niederen Metallalkoholats zugeführt.

Der am Kopf der Kolonne abgezogene Brüden bzw. dessen Kondensat besteht üblicherweise aus im Wesentlichen reinem niederem Alkohol. Das Kondensat kann ohne weitere Aufreinigung ausgeführt und beispielsweise der Herstellung von niederen Metallalkoholaten oder Lösungen davon zugeführt werden.

Die am Sumpf der Kolonne und/oder aus dem Sumpfumlauf abgezogene Lösung des höheren Metallalkoholats besteht üblicherweise im Wesentlichen aus dem höheren Alkohol und dem höheren Metallalkoholat. Die Lösung des höheren Metallalkoholats kann daher als solche weiterverwendet werden, ggf. nach Abkühlen in einem Wärmetauscher.

Alternativ kann der höhere Alkohol vom höheren Metallalkoholat abgetrennt werden. Dies kann durch Eindampfen erfolgen.

Die Erfindung wird durch die beiliegenden Figuren und die nachstehenden Beispiele weiter veranschaulicht.

Fig. 1 zeigt eine zur Herstellung von Metallalkoholaten geeignete Vergleichsanlage.

Fig. 2 zeigt eine zur Herstellung von Metallalkoholaten mittels des erfindungsgemäßen Verfahrens geeignete Anlage.

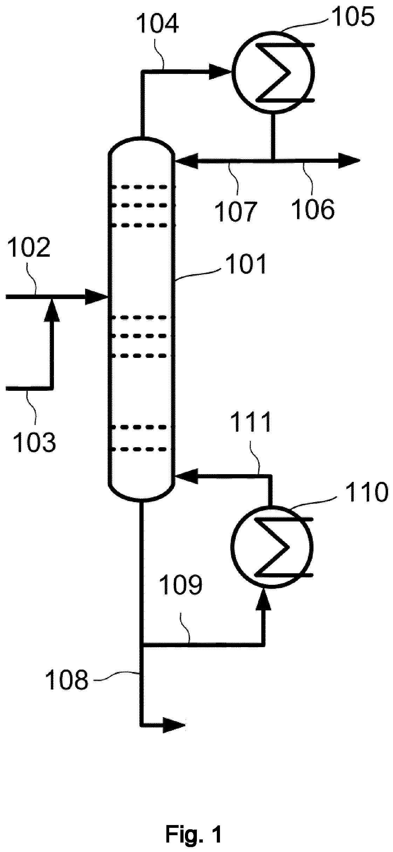

Gemäß Fig. 1 umfasst die Anlage eine Reaktivdestillationskolonne 101. In diese wird über Leitung 102 eine Lösung eines niederen Metallalkoholats zusammen mit einem höheren Alkohol aus Leitung 103 eingespeist.

Am Kopf der Kolonne 101 wird über Leitung 104 ein niederen Alkohol umfassender Brüden abgezogen und in Kondensator 105 kondensiert. Ein erster Teilstrom des kondensierten Brüdens wird über Leitung 106 aus dem Verfahren ausgeführt, während ein zweiter Teilstrom des kondensierten Brüdens über Leitung 107 in den Kopf der Kolonne zurückgeführt wird.

Am Sumpf der Kolonne wird eine Lösung eines höheren Metallalkoholats im höheren Alkohol abgezogen. Ein erster Teilstrom der Lösung des höheren Metallalkoholats wird über Leitung 108 aus dem Verfahren ausgeführt, während ein zweiter Teilstrom der Lösung des höheren Metallalkoholats über den Verdampfer 110 und Leitung 111 in den Sumpf der Kolonne zurückgeführt wird.

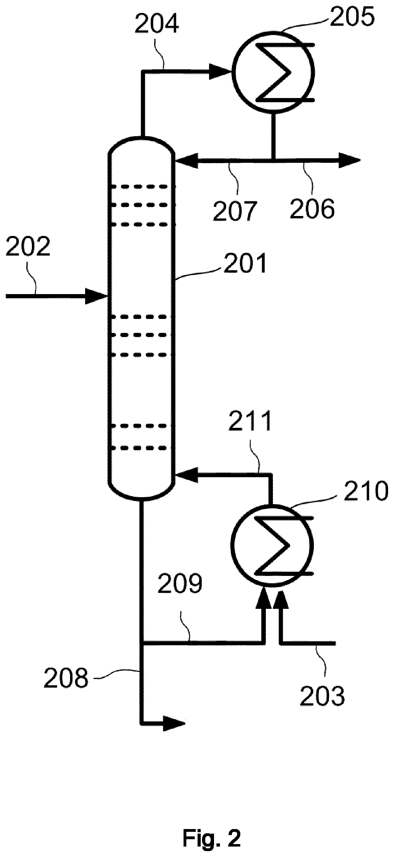

Gemäß Fig. 2 umfasst die Anlage eine Reaktivdestillationskolonne 201. In diese wird über Leitung 202 eine Lösung eines niederen Metallalkoholats eingespeist. Über Leitung 203 und den Verdampfer 210 wird ein höherer Alkohol in den Sumpfumlauf eingespeist.

Am Kopf der Kolonne 201 wird über Leitung 204 ein niederen Alkohol umfassender Brüden abgezogen und in Kondensator 205 kondensiert. Ein erster Teilstrom des kondensierten Brüdens wird über Leitung 206 aus dem Verfahren ausgeführt, während ein zweiter Teilstrom des kondensierten Brüdens über Leitung 207 in den Kopf der Kolonne zurückgeführt wird.

Am Sumpf der Kolonne wird eine Lösung eines höheren Metallalkoholats im höheren Alkohol abgezogen. Ein erster Teilstrom der Lösung des höheren Metallalkoholats wird über Leitung 208 aus dem Verfahren ausgeführt, während ein zweiter Teilstrom der Lösung des höheren Metallalkoholats über den Verdampfer 210 gemeinsam mit dem höheren Alkohol aus Leitung 203 über Leitung 211 in den Sumpf der Kolonne zurückgeführt wird.

### Beispiele

Es wurden Versuche zur Herstellung von Natriumalkoholaten mittels Umalkoholisieren aus Natriummethanolat und höheren Alkoholen durchgeführt, wobei Isopropanol, 2-Butanol bzw. 2-Methyl-2-butanol als höhere Alkohole verwendet wurden.

### Methoden

### A. Bestimmung der Konzentration des höheren Alkohols im Kopfkondensat

Zur Bestimmung des Gehalts an höherem Alkohol im Kopfkondensat wurde eine Probe entnommen, 1,4-Dioxan als interner Standard zugegeben und die Probe mittels Gaschromatographie hinsichtlich ihres Alkoholgehalts analysiert (Trennsäule RTX-5 Amin, Länge 30 m, Innendurchmesser 0,32 mm, Filmdicke 1,5 µm). Die Nachweisgrenze betrug ca. 500 mg/kg.

### B. Bestimmung der Konzentration an Methanol im Sumpfaustrag

### B.1 Höherer Alkohol: Isopropanol oder 2- Butanol

Zur Bestimmung des Methanol-Gehalts im Sumpf der Kolonne wurden bei der Verwendung von Isopropanol als höherer Alkohol 150 mg, bei der Verwendung von 2-Butanol 60 mg einer Probe in ein 22,5 mL Headspace-Glas eingewogen.

Die Probe wurde mit 1 mL Leitungswasser versetzt, mit einer Aluminiumkappe gasdicht verschlossen und per Headspace-GC analysiert (Trennsäule DB-1, Länge 30 m, Innendurchmesser 0,25 mm, Filmdicke 1,0 µm). Die Quantifizierung erfolgte mittels des Standard-Additionsverfahrens. Die Nachweisgrenze betrug weniger als 100 mg/kg.

Beim Standard-Additionsverfahren wird eine Mehrfachbestimmung der Probe durchgeführt, beispielsweise eine Doppelbestimmung. Dabei wird jeder Probe mehrfach eine spezifische Menge der zu bestimmenden Substanz (des höheren Alkohols) hinzugefügt und die Probe nach jeder Zugabe gemessen. Der Zuwachs des Substanzsignals wird ermittelt. Die Konzentration des höheren Alkohols in der ursprünglichen Probe kann durch lineare Regression berechnet werden.

Die Löslichkeit der Proben muss vorab geprüft werden. Bilden sich zwei Phasen aus, muss die Einwaage reduziert werden.

### B.2 Höherer Alkohol: 2-Methyl-2-butanol

Zur Bestimmung des Methanol-Gehalts im Sumpf der Kolonne wurden 500 mg einer Probe entnommen, welche man auf Raumtemperatur abkühlen ließ. Die Probe wurde mit 1 mL Wasser und 0,5 mg tert-Butanol in Dioxan (1 mL, als interner Standard) vermengt, mit einem Tropfen Phosphorsäure versetzt und mit 3 mL Dioxan (ohne internen Standard) verdünnt, um eine verdünnte Probe zu erhalten. Im Falle fester Proben wurden diese vor dem Vermischen mit Wasser, tert-Butanol, Phosphorsäure und Dioxan bei 60 °C aufgeschmolzen. Die verdünnte Probe wurde mittels Gaschromatographie hinsichtlich ihres Alkoholgehalts analysiert (Trennsäule DB-1, Länge 30 m, Innendurchmesser 0,25 mm, Filmdicke 1,0 µm). Die Nachweisgrenze betrug ca. 200 mg/kg.

### C. Bestimmung der Konzentration an Alkoholat im Sumpfaustrag

Zur Bestimmung des Alkoholats im Sumpf der Kolonne wurde eine Probe entnommen und mittels Titration in 2-Propanol mit Trifluormethansulfonsäure (0,1 mol/l in 2-Propanol) der Gesamtgehalt an Basen bestehend aus Alkoholat, Hydroxiden und Carbonat bestimmt. Die Menge an Hydroxiden und Carbonat wurde mittels volumetrischer Karl-Fischer-Titration (KFT) bestimmt, da diese Bestandteile in der KFT mit den KF-Komponenten reagieren und Wasser bilden. Der Beitrag von Hydroxiden und Carbonaten wird vom Gesamtgehalt an Basen subtrahiert, um den Gehalt an Alkoholat zu ermitteln.

### Beispiele 1 bis 5 und Vergleichsbeispiele 1 bis 3

Die Beispiele wurden in einer Anlage durchgeführt, welche eine Glockenbodenkolonne aus Glas mit 80 Böden und einem Zwangsumlaufentspannungsverdampfer umfasste. In den Tabellen 1A und 1B sind die spezifischen Parameter der Beispiele gezeigt. Der Verdampfer wurde mit einem handelsüblichen Thermostat (Julabo HT6) mit einer maximalen Heizleistung von 5700 W beheizt. Der Durchmesser der Kolonne betrug 50 mm. Natriummethanolat (30 Gew.-% in Methanol) wurde seitlich in die Kolonne eingespeist. Zur Vermeidung von Wärmeverlusten wurde die Kolonne mit einer elektrischen Schutzheizung isotherm beheizt.

Der höhere Alkohol wurde entweder vor dem Verdampfer oder auf einen Boden im Abtriebsteil oder zusammen mit Natriummethanolat seitlich in die Kolonne eingespeist. Die Menge an höherem Metallalkoholat bzw. Methanol in der am Sumpf abgezogenen Lösung des höheren Metallalkoholats wurde bestimmt.

Am Kopf der Kolonne wurde der Brüden ausgeführt und in einem Kondensator kondensiert. Die Menge an höherem Alkohol im Kopfkondensat wurde bestimmt.

Vergleichsbeispiel 1 wurde zunächst wie Beispiel 1 durchgeführt. Nach Einstellung des stationären Zustandes wurde die Einspeisung von Isopropanol vor dem Verdampfer eingestellt und Isopropanol stattdessen zusammen mit Natriummethanolat eingespeist. Die Zusammensetzung des Kopfkondensats bzw. des am Sumpf abgezogenen Natriumisopropanolats wurde nach erneutem Einstellen des stationären Zustandes bestimmt.

Vergleichsbeispiel 2 wurde zunächst wie Beispiel 2 durchgeführt. Nach Einstellung des stationären Zustandes wurde die Einspeisung von sec-Butanol vor dem Verdampfer eingestellt und sec-Butanol stattdessen zusammen mit Natriummethanolat eingespeist. Die Zusammensetzung des Kopfkondensats bzw. des am Sumpf abgezogenen Natrium-sec-butanolats wurde nach erneutem Einstellen des stationären Zustandes bestimmt.

Vergleichsbeispiel 3 wurde analog zu Beispiel 5 durchgeführt, mit dem Unterschied, dass die Einspeisung von 2-Methyl-2-butanol nicht vor dem Verdampfer, sondern zusammen mit Natriummethanolat auf Boden 30 erfolgte. Die Zusammensetzung des Kopfkondensats bzw. des am Sumpf abgezogenen Natrium-2-methyl-2-butanolats wurde nach Einstellen des stationären Zustandes bestimmt.

**Tabelle 1A: Experimentelle Versuchsdaten zu Beispielen 1 und 2 sowie Vergleichsbeispielen 1 und 2.**

| | Beispiel 1 | Vergleichsbeispiel 1 | Beispiel 2 | Vergleichsbeispiel 2 | | | |
|---|---|---|---|---|---|---|---|
| | | | | Variante 1 | Variante 2 | Variante 3 | Variante 4 |
| Höherer Alkohol | Isopropanol | | 2-Butanol | | | | |
| Zulauf Na-Methanolat [kg/h] | 0,23 | 0,23 | 0,49 | 0,49 | 0,49 | 0,49 | 0,49 |
| Zulaufstelle Na-Methanolat | Boden 40 | Boden 40 | Boden 30 | Boden 30 | Boden 30 | Boden 30 | Boden 30 |
| Zulauf des höheren Alkohols [kg/h] | 1,226 | 1,125 | 1,814 | 1,742 | 1,800 | 1,874 | 1,918 |
| Zulaufstelle des höheren Alkohols | Vor Verdampfer | Boden 40 | Vor Verdampfer | Boden 30 | Boden 30 | Boden 30 | Boden 30 |
| Kopfabzug [kg/h] | 0,171 | 0,180 | 0,427 | 0,420 | 0,418 | 0,424 | 0,428 |
| Sumpfabzug [kg/h] | 1,286 | 1,172 | 1,869 | 1,809 | 1,871 | 1,94 | 1,977 |
| Sumpfumlauf [kg/h] | 150 | 150 | 150 | 150 | 150 | 150 | 150 |
| Rücklauf [kg/h] | 1,341 | 1,343 | 0,700 | 0,700 | 0,835 | 0,900 | 1,001 |
| Rücklaufverhältnis | 7,84 | 7,46 | 1,64 | 1,67 | 2,00 | 2,12 | 2,34 |
| Verhältnis Zulauf / Rücklauf | 0,172 | 0,171 | 0,700 | 0,700 | 0,587 | 0,544 | 0,4905 |
| T (Kolonnenkopf) [°C] | 63,0 | 62,2 | 62,2 | 62,6 | 62,1 | 62,2 | 62,3 |
| T (Kolonnensumpf) [°C] | 83,8 | 83,8 | 103 | 103 | 103 | 103 | 103 |
| T (Zulauf) [°C] | 47,9 | 54,5 | 52,5 | 54,0 | 54,0 | 54,0 | 54,5 |
| Kopfdruck [mbar, absolut] | 949 | 949 | 949,5 | 949,5 | 949,5 | 949,6 | 949,4 |
| Differenzdruck der Kolonne [mbar] | 81,3 | 82,2 | 73,8 | 76,2 | 78,2 | 79,8 | 82,5 |
| Alkoholat im Sumpf [Gew.-%] | 7,8 | 9,2 | 14,4 | 14,5 | 14,1 | 13,5 | 13,5 |
| Methanol im Sumpf [Gew.-%] | 0,04 | 2,0 | 0,07 | 0,7 | 0,4 | 0,2 | 0,1 |
| Höherer Alkohol im Kopfkondensat [Gew.-%] | 0,15 | 0,4 | 0,360 | 1,04 | n.n. * | n.n. * | n.n. * |

| | | | | | | | |
|---|---|---|---|---|---|---|---|
| * n.n.: unterhalb der Nachweisgrenze | | | | | | | |

**Tabelle 1B: Experimentelle Versuchsdaten zu Beispielen 3 bis 5 sowie Vergleichsbeispiel 3.**

| | Beispiel 3 | Beispiel 4 | Beispiel 5 | | | Vergleichsbeispiel 3 | |
|---|---|---|---|---|---|---|---|
| | | | Variante 1 | Variante 2 | Variante 3 | Variante 1 | Variante 2 |
| Höherer Alkohol | 2-Butanol | | 2-Methyl-2-Butanol | | | | |
| Zulauf Na-Methanolat [kg/h] | 0,49 | 0,49 | 0,15 | 0,225 | 0,10 | 0,15 | 0,10 |
| Zulaufstelle Na-Methanolat | Boden 30 | Boden 30 | Boden 30 | Boden 30 | Boden 30 | Boden 30 | Boden 30 |
| Zulauf des höheren Alkohols [kg/h] | 1,864 | 1,861 | 1,036 | 1,23 | 0,531 | 0,469 | 0,325 |
| Zulaufstelle des höheren Alkohols | Vor Verdampfer | Boden 15 | Vor Verdampfer | Vor Verdampfer | Vor Verdampfer | Boden 30 | Boden 30 |
| Kopfabzug [kg/h] | 0,430 | 0,432 | 0,139 | 0,187 | 0,091 | 0,099 | 0,068 |
| Sumpfabzug [kg/h] | 1,912 | 1,916 | 1,057 | 1,278 | 0,569 | 0,511 | 0,342 |
| Sumpfumlauf [kg/h] | 150 | 150 | 150 | 150 | 150 | 150 | 150 |
| Rücklauf [kg/h] | 0,860 | 0,840 | 0,835 | 1,000 | 1,000 | 1,330 | 1,000 |
| Rücklaufverhältnis | 2,00 | 1,94 | 7,19 | 5,35 | 14,62 | 10,10 | 19,56 |
| Verhältnis Zulauf / Rücklauf | 0,570 | 0,583 | 0,150 | 0,225 | 0,075 | 0,150 | 0,075 |
| T (Kolonnenkopf) [°C] | 62,4 | 62,3 | 62,7 | 63 | 62,6 | 62,3 | 62,3 |
| T (Kolonnensumpf) [°C] | 103 | 103 | 103,7 | 104,2 | 104,5 | 106,5 | 106,5 |
| T (Zulauf) [°C] | 50,3 | 50,0 | 47,4 | 49,9 | 47,7 | 52,2 | 51 |
| Kopfdruck [mbar, absolut] | 949,5 | 949,5 | 949,3 | 949,3 | 949,1 | 949,8 | 949,9 |
| Differenzdruck der Kolonne [mbar] | 78,7 | 78,2 | 81,8 | 83,7 | 88,9 | 80,6 | 88,1 |
| Alkoholat im Sumpf [Gew.-%] | 13,4 | 13,4 | 9,6 | 12,1 | 10,1 | 16,7 | 17,4 |
| Methanol im Sumpf [Gew.-%] | 0,012 | 0,09 | 0,27 | 0,76 | 0,04 | 0,82 | 0,16 |
| Höherer Alkohol im Kopfkondensat [Gew.-%] | 0,3 | n.n.* | 8,85 | 1,19 | 6,28 | n.n.* | n.n. * |

| | | | | | | | |
|---|---|---|---|---|---|---|---|
| * n.n.: unterhalb der Nachweisgrenze | | | | | | | |

Aus dem Vergleich von Beispiel 1 mit Vergleichsbeispiel 1 ist ersichtlich, dass das Einspeisen des höheren Alkohols vor dem Verdampfer gegenüber dem gemeinsamen Einspeisen des höheren Alkohols mit dem niederen Alkoholat auf Boden 40 der Kolonne bei gleichem Verhältnis von Zulauf zu Rücklauf eine niedrigere Methanol-Konzentration im Sumpf bewirkt.

Aus dem Vergleich von Beispiel 2 mit Vergleichsbeispiel 2 (Variante 1) ist wiederum ersichtlich, dass das Einspeisen des höheren Alkohols vor dem Verdampfer gegenüber dem gemeinsamen Einspeisen des höheren Alkohols mit dem niederen Alkoholat auf Boden 30 der Kolonne bei gleichem Verhältnis von Zulauf zu Rücklauf eine niedrigere Methanol-Konzentration im Sumpf bewirkt. Selbst bei einer Erhöhung des Rücklaufs in Varianten 2 bis 4 des Vergleichsbeispiels 2 von 0,7 kg/h auf 1,0 kg/h sinkt die Methanol-Konzentration im Sumpf lediglich auf 0,1 Gew.-%, gegenüber 0,07 Gew.-% in Beispiel 2.

In Beispiel 3 wurde der höhere Alkohol vor dem Verdampfer eingespeist. In Beispiel 4 wurde der höhere Alkohol im Abtriebsteil auf Boden 15 eingespeist. In Vergleichsbeispiel 2 (Variante 2) wurde der höhere Alkohol zusammen mit dem niederen Alkoholat auf Boden 30 eingespeist. Die Rücklaufverhältnisse waren bei diesen drei Versuchen vergleichbar. Es ist ersichtlich, dass die Methanol-Konzentration im Sumpf in Beispiel 3 und 4 signifikant geringer war als in Vergleichsbeispiel 2 (Variante 2). Dies zeigt, dass das Einspeisen des höheren Alkohols vor dem Verdampfer oder im Abtriebsteil der Kolonne gegenüber dem gemeinsamen Einspeisen des höheren Alkohols mit dem niederen Alkoholat auf Boden 30 der Kolonne bei gleichem Verhältnis von Zulauf zu Rücklauf eine niedrigere Methanol-Konzentration im Sumpf bewirkt. Besonders vorteilhaft ist das Einspeisen des höheren Alkohols vor dem Verdampfer.

Aus dem Vergleich von Beispiel 5, Variante 1 mit Vergleichsbeispiel 3, Variante 1 ist ersichtlich, dass das Einspeisen des höheren Alkohols vor dem Verdampfer gegenüber dem gemeinsamen Einspeisen des höheren Alkohols mit dem niederen Alkoholat auf Boden 30 der Kolonne bei gleichem Verhältnis von Zulauf zu Rücklauf eine niedrigere Methanol-Konzentration im Sumpf bewirkt. Dies ist auch aus dem Vergleich von Beispiel 5, Variante 3 mit Vergleichsbeispiel 3, Variante 2 ersichtlich.

Es wurde ferner für ausgewählte Beispiele anhand der Temperatur des Heizöls (KORASILON Öl M 10; spez. Wärmekapazität cp (120°C): 1,74 kJ/(kg K)) im Vor- bzw. Rücklauf die spezifische Heizleistung des Herstellungsverfahrens bestimmt. Die Ergebnisse sind in Tabelle 2 dargestellt.

**Tabelle 2: Spezifische Heizleistung**

| | Beispiel 2 | Vergleichsbeispiel 2, Variante 4 | Beispiel 5, Variante 2 | Vergleichsbeispiel 3, Variante 1 |
|---|---|---|---|---|
| Methanol im Sumpf [Gew.-%] | 0,07 | 0,1 | 0,76 | 0,82 |
| Öltemperatur Vorlauf [°C] | 120,1 | 122,8 | 122,5 | 127,7 |
| Öltemperatur Rücklauf [°C] | 115,4 | 117,6 | 117,5 | 121,2 |
| ΔT Vorlauf / Rücklauf [°C] | 4,7 | 5,2 | 5 | 6,5 |
| Ölmassenstrom [kg/h] | 331,0 | 322,3 | 319,1 | 190,1 |
| Heizleistung Verdampfer [W] | 752 | 810 | 771 | 597 |
| spezifische Heizleistung [J/g] | 10,1 | 10,9 | 18,0 | 25,2 |

Es ist ersichtlich, dass das erfindungsgemäße Verfahren bei vergleichbarer Trennaufgabe eine geringere spezifische Heizleistung benötigt, als wenn der höhere Alkohol zusammen mit dem niederen Metallalkoholat eingespeist wird.

## Patentansprüche

1. Verfahren zur Herstellung von Metallalkoholaten mittels Umalkoholisieren, wobei man
- ein niederes Metallalkoholat über einen seitlichen Zulauf in eine Reaktivdestillationskolonne mit oberhalb des Zulaufs gelegenem Verstärkungsteil und unterhalb des Zulaufs gelegenem Abtriebsteil einspeist;
- einen höheren Alkohol in den Abtriebsteil, den Sumpf und/oder einen Sumpfumlauf der Kolonne einspeist;
- am Sumpf der Kolonne und/oder aus dem Sumpfumlauf eine Lösung eines höheren Metallalkoholats im höheren Alkohol abzieht; und
- am Kopf der Kolonne einen niederen Alkohol umfassenden Brüden abzieht, den Brüden zumindest teilweise kondensiert und einen Teilstrom des Kondensats als Rücklauf auf den Kopf der Kolonne zurückführt;
wobei der höhere Alkohol einen höheren Siedepunkt aufweist als der niedere Alkohol.

2. Verfahren nach Anspruch 1, wobei der höhere Alkohol in flüssiger Form in den Sumpf und/oder den Sumpfumlauf der Kolonne eingespeist wird.

3. Verfahren nach Anspruch 1 oder 2, wobei die Lösung des höheren Metallalkoholats höchstens 1,0 Gew.-% des niederen Alkohols umfasst, bezogen auf das Gesamtgewicht der Lösung des höheren Metallalkoholats.

4. Verfahren nach einem der vorhergehenden Ansprüche, wobei die Lösung des höheren Metallalkoholats 3 bis 60 Gew.-% des höheren Metallalkoholats umfasst, bezogen auf das Gesamtgewicht der Lösung des höheren Metallalkoholats.

5. Verfahren nach einem der vorhergehenden Ansprüche, wobei die Kolonne einen Zwangsumlaufverdampfer aufweist und man den höheren Alkohol in flüssiger Form in den Zulauf zum Zwangsumlaufverdampfer einspeist.

6. Verfahren nach einem der vorhergehenden Ansprüche, wobei das Kopfkondensat höchstens 0,8 Gew.-% des höheren Alkohols umfasst, bezogen auf das Gesamtgewicht des Kopfkondensats.

7. Verfahren nach einem der vorhergehenden Ansprüche, wobei man das niedere Metallalkoholat als Lösung in dem niederen Alkohol einspeist und die Lösung 20 bis 40 Gew.-% des niederen Metallalkoholats umfasst, bezogen auf das Gesamtgewicht der Lösung des niederen Metallalkoholats.

8. Verfahren nach einem der vorhergehenden Ansprüche, wobei das niedere Metallalkoholat ein Alkalimetallalkoholat ist.

9. Verfahren nach Anspruch 8, wobei das niedere Metallalkoholat ein Natriumalkoholat oder ein Kaliumalkoholat ist.

10. Verfahren nach Anspruch 9, wobei das niedere Metallalkoholat Natriummethanolat oder Kaliummethanolat ist, insbesondere Natriummethanolat.

11. Verfahren nach einem der vorhergehenden Ansprüche, wobei der höhere Alkohol ausgewählt ist unter Isopropanol, sec-Butanol, 2-Methyl-2-butanol, tert-Butanol, 2-Methyl-2-pentanol, 3-Methyl-3-pentanol, 3-Ethyl-3-pentanol, 2-Methyl-2-hexanol und 3-Methyl-3-hexanol, insbesondere unter Isopropanol, 2-Methyl-2-butanol, 3-Methyl-3-pentanol und 3-Ethyl-3-pentanol.

## Claims

1. A process for preparing metal alkoxides by means of transalcoholization, wherein
- a lower metal alkoxide is fed via a side feed into a reactive distillation column comprising a rectifying section situated above the feed and a stripping section situated below the feed;
- a higher alcohol is fed into the stripping section, the bottom and/or a bottoms circuit of the column;
- a solution of a higher metal alkoxide in the higher alcohol is taken off at the bottom of the column and/or from the bottoms circuit; and
- a vapor comprising lower alcohol is taken off at the top of the column, the vapor is at least partially condensed and a substream of the condensate is recycled to the top of the column as reflux;
wherein the higher alcohol has a higher boiling point than the lower alcohol.

2. The process according to claim 1, wherein the higher alcohol is fed into the bottom and/or the bottoms circuit of the column in liquid form.

3. The process according to claim 1 or 2, wherein the solution of the higher metal alkoxide comprises not more than 1.0% by weight of the lower alcohol, based on the total weight of the solution of the higher metal alkoxide.

4. The process according to any of the preceding claims, wherein the solution of the higher metal alkoxide comprises 3% to 60% by weight of the higher metal alkoxide, based on the total weight of the solution of the higher metal alkoxide.

5. The process according to any of the preceding claims, wherein the column has a forced circulation evaporator and the higher alcohol is fed in liquid form into the feed to the forced circulation evaporator.

6. The process according to any of the preceding claims, wherein the top condensate comprises not more than 0.8% by weight of the higher alcohol, based on the total weight of the top condensate.

7. The process according to any of the preceding claims, wherein the lower metal alkoxide is fed in as a solution in the lower alcohol and the solution comprises 20% to 40% by weight of the lower metal alkoxide, based on the total weight of the solution of the lower metal alkoxide.

8. The process according to any of the preceding claims, wherein the lower metal alkoxide is an alkali metal alkoxide.

9. The process according to claim 8, wherein the lower metal alkoxide is a sodium alkoxide or a potassium alkoxide.

10. The process according to claim 9, wherein the lower metal alkoxide is sodium methoxide or potassium methoxide, especially sodium methoxide.

11. The process according to any of the preceding claims, wherein the higher alcohol is selected from isopropanol, sec-butanol, 2-methyl-2-butanol, tert-butanol, 2-methyl-2-pentanol, 3-methyl-3-pentanol, 3-ethyl-3-pentanol, 2-methyl-2-hexanol and 3-methyl-3-hexanol, especially from isopropanol, 2-methyl-2-butanol, 3-methyl-3-pentanol and 3-ethyl-3-pentanol.

## Revendications

1. Procédé de fabrication d'alcoolates métalliques par transalcoolisation, dans lequel
- on injecte par une alimentation latérale un alcoolate métallique inférieur dans une colonne de distillation réactive comportant une section de rectification disposée au-dessus de l'alimentation et une section d'épuisement disposée en dessous de l'alimentation ;
- on injecte un alcool supérieur dans la section d'épuisement, dans le fond et/ou dans une recirculation du fond de la colonne ;
- on soutire au fond de la colonne, et/ou de la recirculation du fond, une solution d'un alcool métallique supérieur dans l'alcool supérieur ; et
- on soutire en tête de la colonne une vapeur comprenant un alcool inférieur, on condense au moins partiellement la vapeur et on renvoie un courant partiel du condensat sous forme de reflux en tête de la colonne ;
dans lequel l'alcool supérieur présente un point d'ébullition supérieur à celui de l'alcool inférieur.

2. Procédé selon la revendication 1, dans lequel l'alcool supérieur est injecté sous forme liquide dans le fond et/ou dans la recirculation du fond de la colonne.

3. Procédé selon la revendication 1 ou 2, dans lequel la solution de l'alcoolate métallique supérieur comprend au plus 1,0 % en poids de l'alcool inférieur, par rapport au poids total de la solution de l'alcoolate métallique supérieur.

4. Procédé selon l'une des revendications précédentes, dans lequel la solution de l'alcoolate métallique supérieur comprend 3 à 60 % en poids de l'alcoolate métallique supérieur, par rapport au poids total de la solution de l'alcoolate métallique supérieur.

5. Procédé selon l'une des revendications précédentes, dans lequel la colonne comprend un évaporateur à circulation forcée, et on injecte l'alcool supérieur sous forme liquide dans l'alimentation de l'évaporateur à circulation forcée.

6. Procédé selon l'une des revendications précédentes, dans lequel le condensat de tête comprend au plus 0,8 % en poids de l'alcool supérieur, par rapport au poids total du condensat de tête.

7. Procédé selon l'une des revendications précédentes, dans lequel on injecte l'alcoolate métallique inférieur sous forme d'une solution dans l'alcool inférieur, et la solution comprend 20 à 40 % en poids de l'alcoolate métallique inférieur, par rapport au poids total de la solution de l'alcoolate métallique inférieur.

8. Procédé selon l'une des revendications précédentes, **caractérisé en ce que** l'alcoolate métallique inférieur est un alcoolate d'un métal alcalin.

9. Procédé selon la revendication 8, dans lequel l'alcoolate métallique inférieur est un alcoolate de sodium ou un alcoolate de potassium.

10. Procédé selon la revendication 9, dans lequel l'alcoolate métallique inférieur est le méthanolate de sodium ou le méthanolate de potassium, en particulier le méthanolate de sodium.

11. Procédé selon l'une des revendications précédentes, dans lequel l'alcool supérieur est choisi parmi les composés isopropanol, sec-butanol, 2-méthyl-2-butanol, tert-butanol, 2-méthyl-2-pentanol, 3-méthyl-3-pentanol, 3-éthyl-3-pentanol, 2-méthyl-2-hexanol et 3-méthyl-3-hexanol, en particulier isopropanol, 2-méthyl-2-butanol, 3-méthyl-3-pentanol et 3-éthyl-3-pentanol.
